# EUROPEAN PATENT APPLICATION

(11) **EP 4 497 434 A1**
(43) Date of publication of application: **29.01.2025**
(21) Application number: 24181132.2
(22) Date of filing: 10.06.2024
(51) Int. Cl.: A61K 9/20, A61K 9/28, A61K 31/7042, A61K 31/7048

(54) **A FILM COATED TABLET FORMULATION COMPRISING EMPAGLIFLOZIN**

(30) Priority: 26.07.2023 TR 202308816
(71) Applicant: Sanovel Ilac Sanayi ve Ticaret A.S., Sisli - Istanbul (TR)
(72) Inventor: SUNEL, Fatih, Istanbul (TR); PEHLIVAN AKALIN, Nur, Istanbul (TR); ARMUT, Merve, Istanbul (TR); ARI, Busra, Istanbul (TR)
(74) Representative: Genç Ilhan, Oznur

(57) **Abstract**

The present invention relates to a film coated tablet comprising Empagliflozin or a pharmaceutically acceptable salt thereof wherein the film coating comprising hydroxypropyl methylcellulose. Furthermore, the tablet is also obtained by using an effective process which is simple, rapid, cost effective, time-saving and industrially convenient process and is created to eliminate the disadvantages of active ingredient

## Description

### Field of the Invention

The present invention relates to a film coated tablet comprising Empagliflozin or a pharmaceutically acceptable salt thereof wherein the film coating comprising hydroxypropyl methylcellulose. Furthermore, the tablet is also obtained by using an effective process which is simple, rapid, cost effective, time-saving and industrially convenient process and is created to eliminate the disadvantages of active ingredient.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2: Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose. In Type 2 diabetes, the pancreas makes insulin, but it either doesn't produce enough, or the insulin does not work properly. This diabetes occurs most often in people who are over 40 years old and overweighed. Type 2 diabetes may sometimes be controlled with a combination of diet, weight management, and exercise. However, treatment also may include oral glucose-lowering medications or insulin injections.

Empagliflozin is a known SGLT2 inhibitor that is described for the treatment or improvement in glycemic control in patients with type 2 diabetes mellitus. The chemical name of empagliflozin is 1 - chloro-4-(3-D-glucopyranos-1 -yl)- 2-[4-((S)-tetrahydrofuran-3-yloxy)-benzyl]-benzene and its chemical structure is shown in the Formula I.

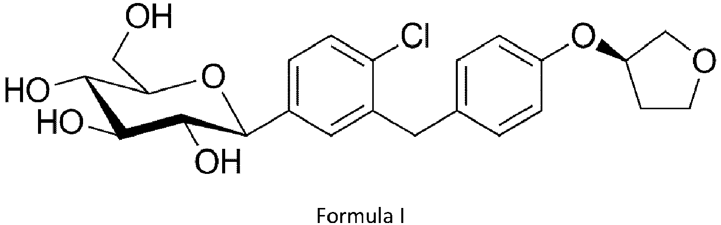

Empagliflozin is available on the market in the form of a free base and is sold under trade name Jardiance<^{®}>, as an oral tablet in 10 mg and 25 mg strengths. Further it is available on the market as a combination product with Metformin and a combination product with Linagliptin.

Active ingredients have some disadvantages in the formulation and process. The main problem encountered when preparing formulations comprising empagliflozin is low solubility, leading to difficulties with disintegration and dissolution profile.

Patent CN102387783A improves the dissolution rate of empagliflozin by controlling the particle size distribution of the raw material and the particle size of the drug particles, but the preparation method is not effective, and the loss of active agent is large, and it is easy to agglomerate and agglomerate after the micro powder, which virtually reduces the production efficiency, so cause stability problems.

Patent CN104623684A prepares mannitol and empagliflozin into a solid dispersion through solid dispersion technology to improve the dissolution rate.

In the prior art, several patents have been done for the dissolution profile. However, since empagliflozin is an active ingredient that has stability problems and is used in small amounts in formulation, focusing only on its dissolution profile will not yield successful results. Therefore, formulation and coating have gained importance.

There still remains a need in the art to provide an improved a film coated tablet comprising empagliflozin having excellent pharmacochemical properties, long-term stability. In the present invention, the film coated tablet is created to overcome the above problems and provides additional advantages to the relevant field of art. Other advantages and embodiments of the present invention will be clarified in the following description.

### Detailed Description of the Invention

The main object of the present invention is to provide a desired stability of the film coated tablet comprising empagliflozin or a pharmaceutically acceptable salt thereof.

Another object of the present invention is to provide a film coated tablet comprising empagliflozin or a pharmaceutically acceptable salt thereof with the desired dissolution profile.

Another object of the present invention is to provide a film coated tablet comprising empagliflozin or a pharmaceutically acceptable salt thereof with excellent physicochemical properties, such as compressibility, flowability, homogeneity, and content uniformity which overcomes the above-described problems in the prior art and have additive advantages over them.

Another object of the present invention is to provide a process for preparing a film coated tablet comprising empagliflozin or a pharmaceutically acceptable salt thereof. The process is wet granulation, it is a simple, rapid, cost effective, time-saving, and industrially convenient method.

Empagliflozin is poorly soluble in water. This causes problems of dissolution profile and bioavailability. The problems negatively affect the stability.

In film coating, coating ingredients of tablets is critical to ensure the stability of the final product. In addition, coating process using excipients can also reduce drug degradation by hydrolysis. We have found that by using an HPMC-based coating, we have found that the stability problem is solved, especially in empagliflozin compositions that have moisture problems. In the film coating tablet in this invention, a hydroxypropyl methylcellulose is used as a film base.

The essence of the invention is to film-coat the core tablet with a coating comprised of the hydroxypropyl methylcellulose, so that the coating simultaneously serves the two purposes of stabilizing the empagliflozin and forming a polymeric film coating around the core.

According to one embodiment of this invention, a film coated tablet comprising Empagliflozin or a pharmaceutically acceptable salt thereof wherein the film coating comprising hydroxypropyl methylcellulose.

According to one embodiment of this invention, the film coating further comprises titanium dioxide.

According to one embodiment of this invention, the film coating further comprises talc or polyethylene glycol or mixtures thereof. The use of peg provides an easy-to-process coating. Its use together with talc provides good stability in the coating and therefore in the tablet.

Empagliflozin is poorly soluble in water. This causes problems of dissolution profile and bioavailability. In the present invention, some studies have been done so that these problems do not appear. At the present invention, thanks to using micronized empagliflozin, the film coated tablet which has a low solubility of empagliflozin or a pharmaceutically acceptable salt thereof or crystalline polymorph thereof has been developed with the desired dissolution profile. Also, this provides excellent pharmacochemical properties of the tablet.

The term "Dx" as used herein means that x% of the particles in a composition (based on volume) have a diameter of or below a specified d value. D (0.9)" or "d90" means that the size at which %90 by volume of the particles are finer. The volume mean particle size of the compound of the Empagliflozin is determined using Malvern Mastersizer 2000 laser diffraction particle size analyzer.

According to one embodiment of this invention, Empagliflozin or a pharmaceutically acceptable salt thereof has a d (0.9) particle size between 1 µm and 5 µm. These particle size helps to provide the desired content uniformity.

According to this embodiment, Empagliflozin or a pharmaceutically acceptable salt thereof has a d (0.9) particle size between 1 µm to 4 µm, between 1 µm to 3 µm.

According to an embodiment of the present invention, the amount of empagliflozin is 2.0% to 20.0% by weight in the total composition. Preferably, the amount of empagliflozin is 3.0% to 15.0% by weight in the total composition.

According to an embodiment of the present invention, empagliflozin is present 10 mg or 25 mg in the composition. Preferably, empagliflozin is present 10 mg in the composition.

According to one embodiment of the present invention, the film coating comprises;
- Opadry Yellow
- Hydroxypropyl methylcellulose
- Titanium Dioxide
- Talc
- PEG
- Iron oxide yellow

According to an embodiment of the present invention, the film coated tablet further comprises at least one pharmaceutically acceptable excipient is selected from the group comprising binders, disintegrants, diluents, lubricants, glidant or mixtures thereof.

Suitable diluents are selected from the group comprising microcrystalline cellulose, anhydrous lactose, lactose, lactose monohydrate, corn starch, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

According to an embodiment of the present invention, at least two diluents are used. The diluents are microcrystalline cellulose and lactose monohydrate. The diluent helps to provide flowability and compressibility of the powder mixture. Also, these diluents help to provide the desired stability.

According to an embodiment of the present invention, the amount of diluents is 65.0% to 92.0% by weight in the total composition.

Flowability is an important parameter in tablet formulation. It can be a problem especially in formulations containing a small amount of active substance. In a formulation, the problem with flowability negatively affects the content uniformity, and the problem with the content uniformity negatively affects the dissolution profile. We have found in this invention that a particularly at least binder overcome this flowability problem.

Suitable binders are selected from the group comprising hydroxypropyl cellulose, sodium carboxymethyl cellulose, polyvinylpyrrolidone, sodium carboxymethyl cellulose, polyethylene glycol, starch, pregelatinized starch, sodium alginate, hydroxypropyl methyl cellulose, carboxy methyl cellulose, methyl cellulose, guar gum, polymethacrylates, methacrylate polymers, hyaluronic acid, pectin, polysaccharides, poloxamer, polyoxyethylene-alkyl ether, polydextrose, polyethylene oxide or mixtures thereof.

According to an embodiment of the present invention, the binder is hydroxypropyl cellulose.

According to an embodiment of the present invention, the amount of binders is 0.5% to 6.0% by weight in the total composition. Preferably, the amount of binders is 1.0% to 5.0% by weight in the total composition.

Suitable disintegrants are selected from the group comprising crospovidone, croscarmellose sodium, low-substituted hydroxypropyl cellulose, carboxymethyl cellulose, sodium carboxymethyl cellulose, calcium carboxymethyl cellulose, sodium carboxymethyl starch, hydroxymethyl starch or mixtures thereof.

According to an embodiment of the present invention, the disintegrant is croscarmellose sodium.

According to an embodiment of the present invention, the amount of disintegrant is 0.5% to 6.0% by weight in the total composition. Preferably, the amount of disintegrant is 1.0% to 4.0% by weight in the total composition.

Suitable lubricant is selected from the group comprising talc, sodium stearyl fumarate, magnesium stearate, potassium stearate, stearic acid, sodium chloride, sodium benzoate, sodium acetate, sodium oleate, polyethylene glycols or mixtures thereof.

According to one embodiment of the present invention, the lubricant is magnesium stearate.

According to one embodiment of the present invention, the amount of lubricant is between 0.1% and 3.0% by weight in the total formulation.

Suitable glidants are selected from the group comprising anhydrous colloidal silicon dioxide, hydrophilic polymers, silicon dioxide or mixtures thereof.

According to one embodiment of the present invention, the glidant is anhydrous colloidal silicon dioxide. This excipient provides the flowability of the powder/granules.

According to one embodiment of the present invention, the amount of glidant is between 0.1% and 3.0% by weight in the total formulation.

According to one embodiment of the present invention, the film coating tablet is free of surfactant. This helps to provide the desired stability.

According to one embodiment of the present invention, the tablet comprises;
- Empagliflozin
- Lactose monohydrate
- Microcrystalline cellulose PH101
- HPC LF
- Croscarmellose sodium
- Anhydrous colloidal silicon dioxide
- Magnesium stearate.

Wet granulation efficiently counteracts the segregation of active agent, so it is observed that the desired stability, flowability and content uniformity.

According to this embodiment of the invention, the film coated tablet of the present invention can be prepared by using wet granulation. So, an easy method was created to eliminate the disadvantages of active ingredient. The desired dissolution profile and a desired homogeneity of the film coated tablet is obtained and it has a simple preparation process, in favor of industrial production.

According to this embodiment of the present invention, a solvent is used at wet granulation.

Suitable solvents are selected from the group comprising pure water, dichloromethane, 0.1N HCl, methanol, ethanol, propylene glycol, polyethylene glycol, glycerine triacetate or mixtures thereof. Preferably, the solvent is water.

According to one embodiment of the present invention, a process for the preparation of the film coated tablet comprises the following steps:
a) Mixing empagliflozin, lactose monohydrate, microcrystalline cellulose and HPC,
b) Adding a solvent (preferably water) and granulating,
c) Sieving the wet granule,
d) Drying the wet granule and obtained dry granule,
e) Sieving the dried granule,
f) Adding croscarmellose sodium and mixing,
g) Adding anhydrous colloidal silicon dioxide and magnesium stearate and then mixing,
h) Compressing the mixture into tablets.
i) Coating the tablets with film coating comprising HPMC.

### Example 1:

| **Ingredients** | **% by weight of the total formulation** |
|---|---|
| Empagliflozin | 2.0 -20.0 |
| Lactose monohydrate | 50.0 - 70.0 |
| Microcrystalline cellulose PH101 | 10.0 - 30.0 |
| HPC LF | 0.5 -6.0 |
| Croscarmellose sodium | 0.5 -6.0 |
| Anhydrous colloidal silicon dioxide | 0.1 -3.0 |
| Magnesium stearate | 0.1 -3.0 |
| **TOTAL** | **100** |

### Example 2:

| **Ingredients** | **% by weight of the total formulation** | | |
|---|---|---|---|
| Empagliflozin | 12.5 | 4.0 | 12.5 |
| Lactose monohydrate | 56.0 | 65.0 | 56.0 |
| Microcrystalline cellulose PH101 | 25.0 | 25.0 | 25.0 |
| HPC LF | 3.0 | 3.0 | 3.0 |
| Croscarmellose sodium | 2.0 | 2.0 | 2.0 |
| Anhydrous colloidal silicon dioxide | 0.5 | 0.5 | 0.5 |
| Magnesium stearate | 0.5 | 0.5 | 0.5 |
| **TOTAL** | **100** | **100** | **100** |

A process for example 1 or 2;
a) Mixing empagliflozin, lactose monohydrate, microcrystalline cellulose and HPC,
b) Adding a solvent (preferably water) and granulating,
c) Sieving the wet granule,
d) Drying the wet granule and obtained dry granule,
e) Sieving the dried granule,
f) Adding croscarmellose sodium and mixing,
g) Adding anhydrous colloidal silicon dioxide and magnesium stearate and then mixing,
h) Compressing the mixture into tablets.
i) Coating the tablets with film coating.

| **Film coating for example 1 or 2** |
|---|
| Opadry Yellow |
| Hypromellose |
| Titanium Dioxide |
| Talc |
| Macrogol |
| Iron oxide yellow |

## Claims

1. A film coated tablet comprising Empagliflozin or a pharmaceutically acceptable salt thereof wherein the film coating comprising hydroxypropyl methylcellulose.

2. The film coated tablet according to claim 1, wherein the film coating further comprising titanium dioxide.

3. The film coated tablet according to claim 1, wherein the film coating further comprising talc or polyethylene glycol or mixtures thereof.

4. The film coated tablet according to claim 1, wherein Empagliflozin or a pharmaceutically acceptable salt thereof has a d (0.9) particle size between 1 µm and 5 µm.

5. The film coated tablet according to claim 1, wherein Empagliflozin is present 10 mg in the composition.

6. The film coated tablet according to claim 1, wherein the film coating comprises;
- Opadry Yellow
- Hydroxypropyl methylcellulose
- Titanium Dioxide
- Talc
- PEG
- Iron oxide yellow

7. The film coated tablet according to claim 1, wherein the film coated tablet further comprises at least one pharmaceutically acceptable excipient is selected from the group comprising binders, disintegrants, diluents, lubricants, glidant or mixtures thereof.

8. The film coated tablet according to claim 7, wherein diluents are selected from the group comprising microcrystalline cellulose, anhydrous lactose, lactose, lactose monohydrate, corn starch, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

9. The film coated tablet according to claim 8, wherein the diluents are microcrystalline cellulose and lactose monohydrate.

10. The film coated tablet according to claim 7, wherein binders are selected from the group comprising hydroxypropyl cellulose, sodium carboxymethyl cellulose, polyvinylpyrrolidone, sodium carboxymethyl cellulose, polyethylene glycol, starch, pregelatinized starch, sodium alginate, hydroxypropyl methyl cellulose, carboxy methyl cellulose, methyl cellulose, guar gum, polymethacrylates, methacrylate polymers, hyaluronic acid, pectin, polysaccharides, poloxamer, polyoxyethylene-alkyl ether, polydextrose, polyethylene oxide or mixtures thereof.

11. The film coated tablet according to claim 10, wherein the binder is hydroxypropyl cellulose.

12. The film coated tablet according to claim 7, wherein disintegrants are selected from the group comprising crospovidone, croscarmellose sodium, low-substituted hydroxypropyl cellulose, carboxymethyl cellulose, sodium carboxymethyl cellulose, calcium carboxymethyl cellulose, sodium carboxymethyl starch, hydroxymethyl starch or mixtures thereof.

13. The film coated tablet according to claim 1, wherein the film coating tablet is free of surfactant.

14. The film coated tablet according to claim 1, wherein the tablet comprises;
- Empagliflozin
- Lactose monohydrate
- Microcrystalline cellulose PH101
- HPC LF
- Croscarmellose sodium
- Anhydrous colloidal silicon dioxide
- Magnesium stearate.
